# EUROPEAN PATENT SPECIFICATION

(11) **EP 3 701 855 B1**
(45) Date of publication and mention of the grant of the patent: **26.02.2025**
(21) Application number: 19160373.7
(22) Date of filing: 01.03.2019
(51) Int. Cl.: A61B 5/00, A61B 5/107

(54) **DIAGNOSTIC SYSTEM FOR THE DETECTION AND MONITORING OF VOLUMETRIC AND PERIMETER INDICATORS OF THE HUMAN BODY**
DIAGNOSESYSTEM ZUR ERKENNUNG UND ÜBERWACHUNG VON VOLUMETRISCHEN UND PERIMETRISCHEN INDIKATOREN DES MENSCHLICHEN KÖRPERS
SYSTÈME DE DIAGNOSTIC POUR LA DÉTECTION ET LA SURVEILLANCE D'INDICATEURS VOLUMÉTRIQUES ET DE PÉRIMÈTRE DU CORPS HUMAIN

(43) Date of publication of application: 02.09.2020
(73) Proprietor: Tecno Medical Innovation Srl, 31015 Conegliano, Treviso (IT)
(72) Inventor: GALEANDRO, Innocente Cataldo, 74121 Taranto (IT); DI PONZIO, Egidio, 74122 Taranto (IT); CICCONE, Marco Matteo, 70124 Bari (IT); RIZZI, Luca Carlo Maria, 73100 Lecce (IT); DE CRISTOFARO, Sarah, 72100 Brindisi (IT)
(74) Representative: De Tullio, Michele Elio

(56) References cited:
- WO-A2-2016/071773
- GB-A- 2 564 243
- US-A1- 2015 216 477
- US-A1- 2016 235 354

## Description

### Field of the invention

The present invention relates to a diagnostic system for the detection, assessing and monitoring volumetric and perimetric indicators of a human body, and portions thereof.
The system operates according to a method not part of the invention.

More specifically, the system is implemented with the purpose of generating a multitude of volumetric indicators and perimetric indicators, morphological indicators and a model of the venous tree of a human body, or a portion thereof, which, by using a dedicated method of use not according to the invention, are suitable for obtaining information on the (muscle and fat) mass and on the (lymphatic and vessel) liquid system of a human body, or portions thereof, and also suitable for diagnosing and monitoring pathologies.

### Present status of the art

Systems and procedures are known in the present status of the art which are used for determining the dimensions of a human body, or of a portion thereof. Systems and methods for assessing and monitoring volumes of a human body, or a portion thereof, are also known, with the purpose of monitoring pathologic statuses, among which the following systems and methods are mentioned:
- Methods wherein an operator manually uses a measuring tape for assessing perimeters of a human body, or a portion thereof, at different heights;
- Methods wherein a patient is immersed in a container containing water and the increase of the water level in the container is subsequently measured;
- Methods based on segmenting of images acquired through computerized tomography apparatuses;
- Systems based on coaxial RX capable of providing a 3D image of whole parts of a body;
- Echographic systems equipped with 3D-ECHO facilities.

Patent documents are known in the present status of the art, including documents GB,2,564,243; WO2016/071773; US2015/216477 and US2016/235354. Document US9878209B2 discloses an apparatus and its respective procedure for generating tridimensional (3D) models which represent the dimensions of a human body, by using one or several depth sensors and depth assessing cameras. Determining the dimensions of a body includes measuring any parts of a body, such as for instance the height of a person, the width of his/her chest, the circumference of his/her waist, the length of an arm thereof, etc. Said measurement can be repeated over time to obtain more sets of data which can subsequently be compared to or coupled together for monitoring any changes in the dimensions of a people's body over time. This document describes the assessment of a first image, which allows to generate a first tridimensional model, then to assess a second image, which allows to generate a second tridimensional model. The two images, hence the two models, are subsequently compared to each other to highlight any dimensional changes over time. However, said known systems and methods feature a number of kinds of drawbacks as follows.

First of all, some of said systems and methods require a long time for collecting data or processing data.

Secondly, some of said systems and methods feature high purchasing, maintenance, and operating costs.

Thirdly, some of said systems and methods feature a poor measurement repeatability and are in any case particularly invasive both because of the position taken by the human body during their use, and because of the administration of radiations.

In addition, many of said known systems feature a high risk of human errors, in that they are "operator-dependent" systems and methods, i.e. systems and methods that are much influenced by operator's ability.

The present invention starts from the recognition of the above-mentioned drawbacks to try and correct them.

An object of the present invention is to provide a system for generating a multitude of volumetric indicators and perimetric indicators suitable for monitoring pathologies, while greatly limiting the recourse to approximation operations, hence featuring a high level of reliability and accuracy in the obtained results.

Another object of the present invention is to provide a system as said above, which can be easily embodied, without entailing particularly significant adaptation operations, with reference to means suitable for assessing volumetric and perimetric data, having characteristics even considerably different from each other.

A further object of the present invention is to provide a system as mentioned above, wherein the different volumetric indicators and perimetric indicators suitable for monitoring pathologies in processing operations are easy to update, so as to optimize reliability and effectiveness of the final assessment and diagnostic result.

On the other hand, an object of the present invention is to provide a system as specified above, which allows to make the measurement of volumetric indicators and perimetric indicators, which will not be operator-dependent, more repeatable.

A concept not part of the invention relating to a method for assessing and monitoring volumes of a human body, or portions thereof, through the system according to the present invention for generating volumetric indicators and perimetric indicators is described .

An object of the present invention is also to provide a system for relating said volumetric indicators and perimetric indicators to morphological indicators and to a model of the venous tree of a human body in order to improve diagnostics, progress, and monitoring of pathologies.

Finally, a further object of the present invention is to make it possible to perform orthostatic volumetric measurements, in time repeatable conditions, the patient being in a standing straight position.

In view of such objects, the present invention provides a diagnostic system for assessing and monitoring volumetric and perimetric indicators of a human body, or portions thereof, whose basic characteristic is the subject of claim 1.

Further advantageous characteristics of said procedure are described in the subsequent dependent claims.

Said claims are to be considered here integrally specified.

### Summary of the invention

In one first aspect, the present invention relates to a diagnostic system for generating a multitude of volumetric indicators and perimetric indicators of a human body, or portions thereof, which are suitable for monitoring pathologies and modifications of muscle and fat masses, and of the liquid system, including the lymphatic and vessel ones, of a human body, as indicated in claim 1.

The diagnostic system according to the present invention comprises:
- means suitable for assessing volumetric and perimetric data,
- electronic processor means operationally connected to said means suitable for assessing volumetric and perimetric data,
- software means operationally connected to said means suitable for assessing volumetric data and said electronic processor means,

- means for positioning a human body operationally connected to said means suitable for assessing volumetric and perimetric data,
- means for lighting a human body connected to said means for positioning a human body and operationally connected to said means suitable for assessing volumetric data and said electronic processor means.

The volumetric indicators and the perimetric indicators described in the present invention are to be considered as numerical indices representative of metabolic processes of a human body which allow to monitor pathologies and modifications of muscle and fat masses, and of the liquid system, including the lymphatic and vessel ones, of a human body.

Generating said indicators and monitoring them over time provides information on the progress of pathologies that involve the (muscle and fat) mass and the liquid system (including the lymphatic and vessel ones). Modifications in the morphology of a human body, or a portion thereof, are also monitored via said volumetric indicators and perimetric indicators.

In a second aspect, is described a concept relating to a method not according to the invention for generating and monitoring volumetric indicators and perimetric indicators of a human body, or portions thereof, by using said diagnostic system, characterized in that it comprises the following steps:
- positioning a human body for assessing volumes and perimeters thereof,
- acquiring anatomical markers by using means suitable for acquiring anatomical markers,
- assessing volumetric data by using means suitable for assessing volumes and perimeters,
- processing this volumetric data by using electronic processor means and software means,
- automatically generating volumetric indicators and perimetric indicators,
- providing a feedback on the status of pathology.

Further characteristics and advantages of the present invention will be better highlighted by looking at the following detailed description of preferred but not exclusive embodiments, which are illustrated for indicative, non-limitative purpose only, by making reference to the attached drawings, wherein:
- Figure 1 is a block diagram showing the main component parts of the system;
- Figure 2 shows an axonometric view of the system and of its main component parts;

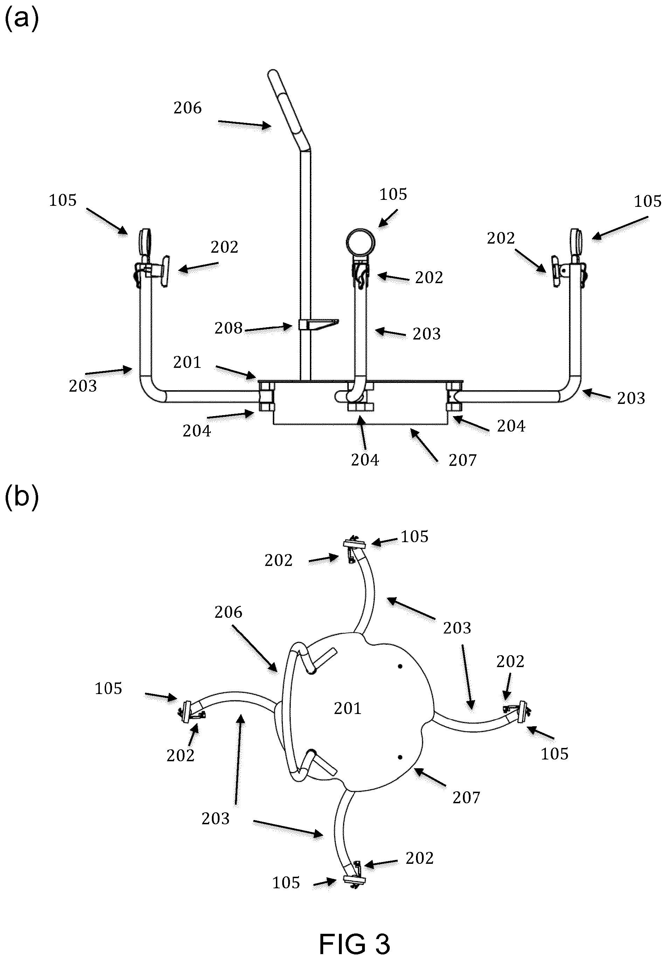

- Figure 3 shows a side view (A) and a top view (B) of the system and of its main component parts;
- Figure 4 shows an axonometric view of the system which illustrates how anatomical markers are acquired in three points;
- Figure 5 shows a view which illustrates a subdivision of a leg into quartiles;
- Figure 6 shows a block diagram of the method not according to the invention;
- Figure 7 shows a block diagram illustrating a variant of the method not according to the invention;
- Figure 8 shows how volumetric indices vary as a function of time in a real case.

### LEGENDA

- 100 - Overall diagnostic system
- 101 - Means suitable for assessing volumetric and perimetric data
- 102 - Electronic processor means
- 103 - Software means
- 104 - Human body positioning means
- 105 - Lighting means
- 201 - Standing base where to lean a first lower limb
- 202 - Cameras
- 203 - Closable arms
- 204 - Point where to secure a closable arm to the footboard
- 205 - Movable laser lines
- 206 - Handle
- 207 - Footboard
- 208 - Point where to lean the second lower limb
- 401a - first marker point or line
- 401b - second marker point or line
- 401c - third marker point or line
- 402 - first lower limb
- 403 - second lower limb
- 501 - Ist quartile of a lower limb
- 502 - IInd quartile of a lower limb
- 503 - Illrd quartile of a lower limb
- 504 - IVth quartile of a lower limb

### Detailed description

The following detailed description relates to specific embodiments of the system of the present invention and a method which is not part of the invention.

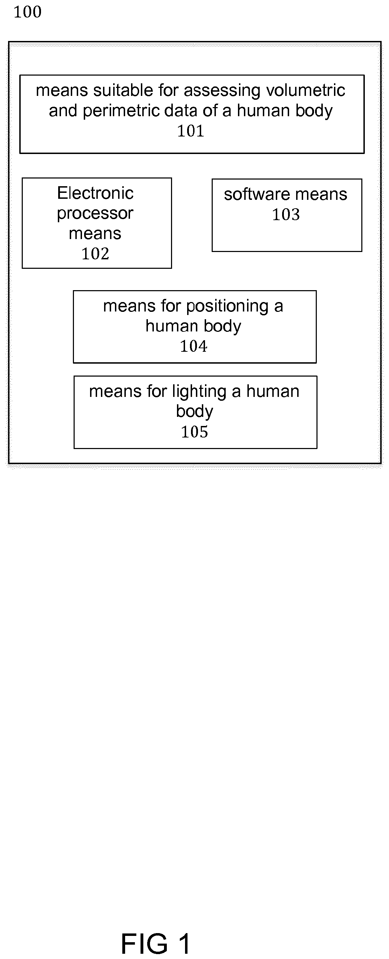

With a specific reference to Figure 1, a diagnostic system 100 is described, comprising:
- means suitable for assessing volumetric and perimetric data 101,
- electronic processor means 102 operationally connected to said means suitable for assessing volumetric and perimetric data 101,
- software means 103 operationally connected to said means suitable for assessing volumetric and perimetric data 101 and said electronic processor means 102,
- means for positioning a human body (104) operationally connected to said means suitable for assessing volumetric and perimetric data (101),
- means for lighting a human body (105) connected to said means for positioning a human body (104) and operationally connected to said means suitable for assessing volumetric and perimetric data (101) and to said electronic processor means (102).

Said system is characterized in that said electronic processor means 102 and said software means 103 process said volumetric and perimetric data and generate a multitude of volumetric indicators and perimetric indicators suitable for monitoring edemas and pathologies included in a group formed of muscle, cardiovascular, venous system, and lymphatic pathologies, over time.

In one first embodiment of the invention said diagnostic system 100 is characterized in that said means suitable for assessing volumetric data and perimetric data 101 are means for acquiring images comprising, jointly or disjointly, video cameras, cameras, scanner, echo-dopplers.

Said means for positioning a human body 104 comprise: a footboard, one or more supports for portions of a human body, handles, standing bases.

Said means for lighting a human body 105 comprise lamps featuring a high chromatic yield index and light diffusers suitable for opening the light beam and for reducing the presence of direct light and shadows. Said means for lighting a human body 105 might comprise LED lamps.

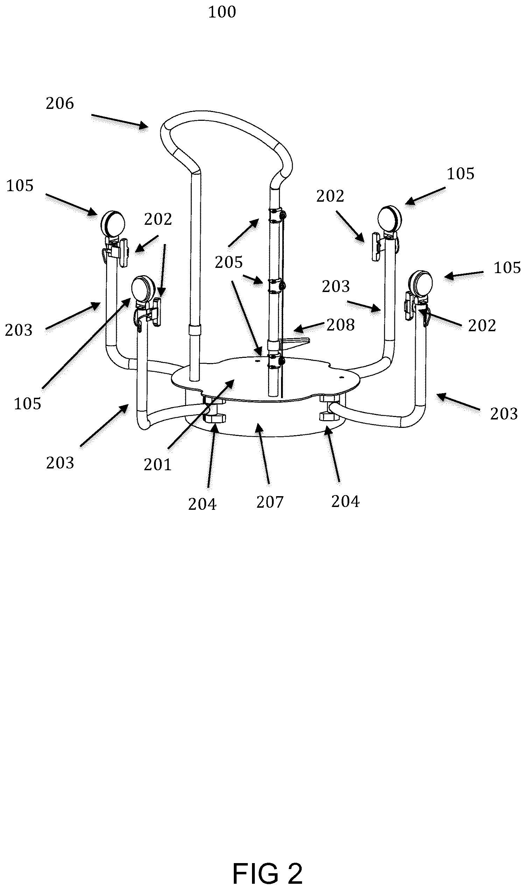

With reference to Figure 2 , said video cameras 202 possibly comprise one or several depth sensors. Conveniently said video cameras 202, comprising depth sensors, are integrally connected to means for positioning a human body 104, in particular they are connected, via folding movable arms 203, to a footboard 207 in junction points 204, wherein every arm 203 accommodates one or more video cameras 202. Advantageously said depth sensors accommodated in said video cameras 202 comprise infrared depth sensors.

In its first embodiment, the present invention is characterized in that said means suitable for assessing volumetric and perimetric data 101, comprising video cameras 202, also include movable laser lines 205 suitable for identifying and using marker points and lines 401a-c.

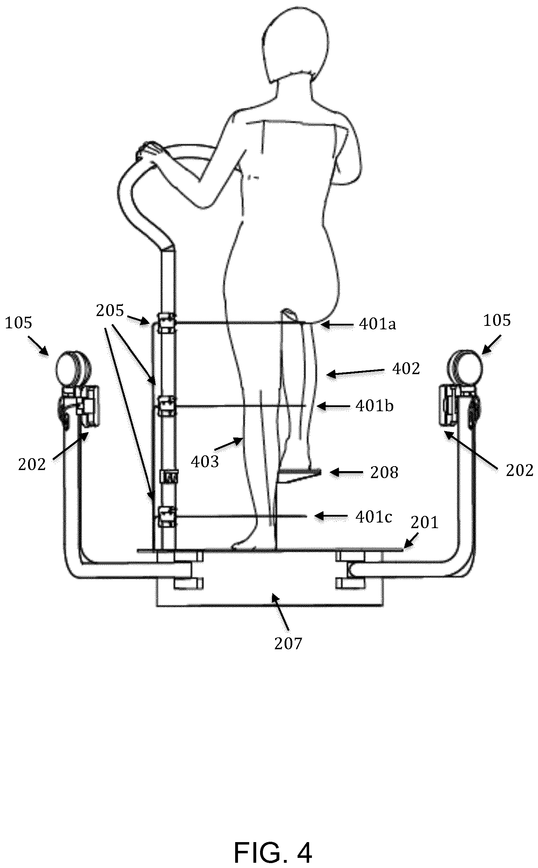

Said means suitable for assessing volumetric and perimetric data 101 and said means for positioning a human body 104, being part of the diagnostic system 100, are for instance applied for generating a multitude of lower limb volumetric indicators and perimetric indicators. With reference to Figure 4 , said means suitable for assessing volumetric and perimetric data 101 are characterized in that they include three movable laser lines 205 suitable for identifying and using marker points or lines 401a-c. Advantageously said marker points or lines 401a-c are positioned at the groin, femoral condyles, and malleolus-ankle levels respectively. In one embodiment of the present invention, the human body is in a standing straight position. The first lower limb 402 is lifted and leans on a support point 208, as shown in Figure 4 , said support point 208 being adjustable in height. The second lower limb 403 is put in contact with the standing base 201 (as is shown in Figure 4 ) of the footboard 207. The position of the first lower limb 402 being raised makes it easily accessible a collection of said volumetric and perimetric data of the inner thigh and improves the quality of the assessment of said data. The positions of the first lower limb 402 and of the second lower limb 403 are interchangeable on the support point 208 and on the standing base 201 of the footboard 207.

In a second embodiment of the invention, said diagnostic system 100 is characterized in that advantageously said means suitable for assessing volumetric data and perimetric data 101 also comprise means suitable for acquiring volumetric data and blood vessel flow data (not shown in the figures). Said means suitable for acquiring volumetric data and blood vessel flow data comprise, as an example, an echo-doppler or an echo-color doppler, operationally connected to said electronic processor means 102, to said software means 103, and to said means for positioning a human body 104. In this second embodiment of the invention, said means suitable for acquiring volumetric data and blood vessel flow data are suitable for generating morphological indicators and a model of the venous tree of a human body, or a portion thereof. With a special reference to the lower limbs 402 - 403, said model of the venous tree is advantageously classified on the basis of eleven standard models of the venous tree or phenotypes. Said phenotypes are a classification of various typologies of chronic venous insufficiency of the lower limbs, a pathological situation wherein an increase in the volume of the limbs takes place, related to a deficit of the drainage system constituted by veins.

Surprisingly a correlation was identified between the model of the venous tree of the lower limbs and the volumetric and perimetric indicators of the lower limbs. In particular, the specific model of the venous tree as determined via the diagnostic system 100, i.e. the specific phenotype, characterizes each individual human body. The evolution of said volumetric and perimetric indicators generated for each individual human body over time is related to the specific phenotype of each individual human body, via a statistical relationship, hence they allow an improved monitoring over time of edemas and pathologies falling in the group formed of muscle, cardiovascular, venous system, and lymphatic system pathologies.

Said monitoring over time is made, for example, on edemas and pathologies of the lower limbs. It has been found that the best therapy applicable to the treatment of edemas and pathologies of the lower limbs can be defined on the basis of the phenotypes, and therapeutic effectiveness can be measured by monitoring said volumetric indicators.

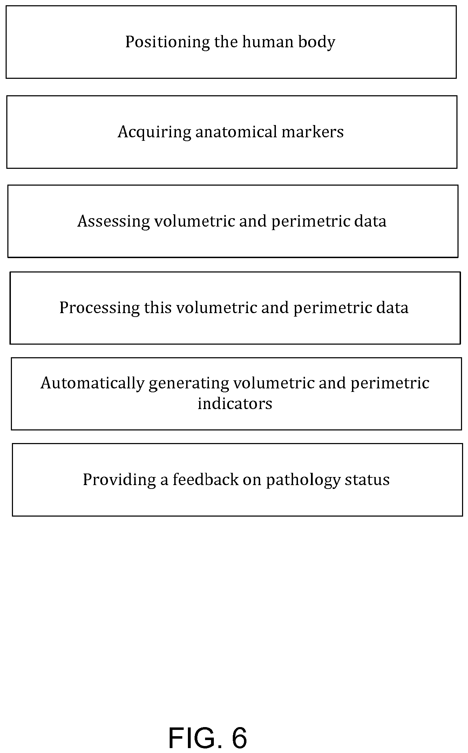

With a special reference to Figure 6, a method is here described for assessing and monitoring volumetric indicators and perimetric indicators of a human body, or parts thereof, determined according to the system 100, and characterized in comprising the following steps:
- positioning a human body for assessing volumes and perimeters thereof,
- acquiring anatomical markers by using means suitable for acquiring anatomical markers,
- assessing volumetric and perimetric data by using means suitable for assessing volumes and perimeters,
- processing this volumetric and perimetric data by using electronic processor means and software means,
- automatically generating volumetric indicators and perimetric indicators,
- providing a feedback on the status of pathology on the basis of said volumetric indicators and perimetric indicators.

In one of its embodiments, the present invention comprises the steps of generating and monitoring volumetric indicators and perimetric indicators for the lower limbs. Advantageously the assessment of said volumetric and perimetric data by using means suitable for assessing volumes and perimeters is made with the help of several video cameras, the human body being in a standing straight position. Said assessment of said volumetric and perimetric data is made, for instance, by acquiring, in a rapid succession, several images from four video cameras 202 positioned in a circular manner about the lower limb thus obtaining several views of one and the same lower limb. Said video cameras 202 include one or several infrared depth sensors and are integrally connected to said means for positioning a human body 104, in particular they are connected, via folding movable arms 203, to a footboard 207 at junction points 204, wherein every arm 203 accommodates one or several video cameras 202.

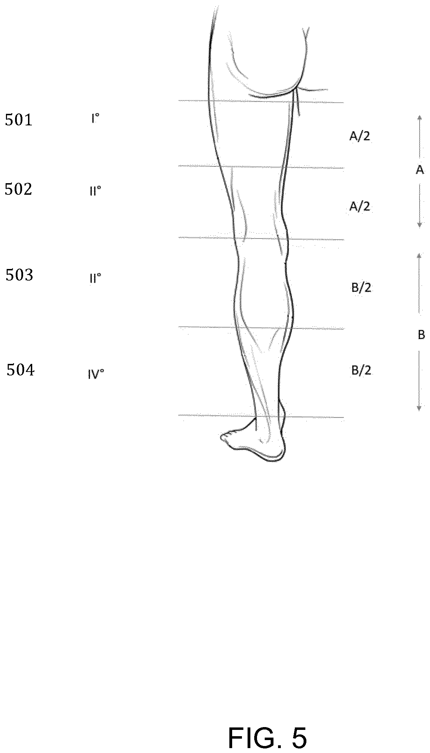

Said volumetric and perimetric data processing by using electronic processor means and software means is made on the images acquired from each of the four video cameras 202, i.e. by processing several views of one and the same lower limb acquired in a rapid succession. Said software means apply image processing and transformation algorithms in order to perform a superimposition of said images, and to apply a filter for cleaning up the images themselves, so as to obtain a cloud of points which characterizes and represents the lower limb. Said software means also make a segmentation of the cloud of points of the lower limb at several cutting levels, i.e. several cutting planes. For every cutting plane of the cloud of points of the lower limb, said software means define a projection of said closest points on the plane, thereby drawing up a polyline which plots the perimeter of the lower limb at the assessment height of interest. The area enclosed by said polyline is subsequently calculated by the software means, and the volume is determined in correspondence with the height of the plane defined for segmentation. The thickness of said plane is, for example, 1 cm. In one embodiment of its own, the method described in the present invention is characterized in that the generation of volumetric and perimetric indicators is made, the lower limbs being subdivided into quartiles, by generating one global volumetric indicator of the limb and one volumetric indicator for each of said quartiles of the limb. Figure 5 shows an example of such subdivision of a lower limb into quartiles: Ist quartile 501, IInd quartile 502, IIIrd quartile 503, IVth quartile 504. The length of the Ist quartile 501 and that of the IInd quartile 502 equal a value corresponding to A/2. The length of the Illrd quartile 503 and that of the IVth quartile 504 equal a value corresponding to B/2. The parameters A and B represent segments of said lower limb, A+B defining the length of the limb from the groin to the malleolus.

Just as an example, said volumetric indicators generated by said electronic processor means and said software means can equal values falling in the following ranges, cubic centimeter (cc) being taken as measuring unit:
- Global volumetric indicator (complete lower limb): 6,000 cc to 11,500 cc;
- Ist quartile volumetric indicator (the highest part of the lower limb, just below the groin): 3,800 cc to 5,500 cc;
- IInd quartile volumetric indicator: 1,300 cc to 3,000 cc;
- Illrd volumetric indicator: 1,000 cc to 2,000 cc;
- IVth quartile volumetric indicator (the lower part of the lower limb, above the malleolus): 500 cc to 900 cc.

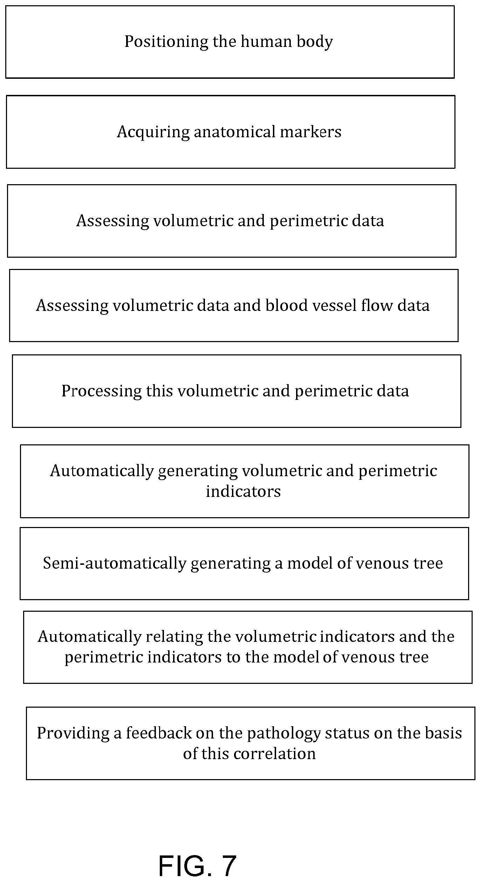

In a further embodiment of the present invention, the method for generating and monitoring volumetric indicators and perimetric indicators of a human body, or parts thereof, as determined according to the system 100, is characterized in comprising the following additional steps ( Figure 7 ):
- assessing data on blood vessels of a human body, or a portion thereof, by using means suitable for acquiring volumetric data and blood vessel flow data,
- processing this volumetric data and blood vessel flow data by using electronic processor means and software means,
- semi-automatically generating a model of the venous tree on the basis of the processed volumetric data and blood vessel flow data,
- automatically relating the volumetric indicators and perimetric indicators to the model of the venous tree,
- providing a feedback on the status of pathology on the basis of the correlation between said volumetric indicators and perimetric indicators and the model of the venous tree.

Advantageously the assessment of said data on blood vessels of a human body, or a portion thereof, by using means suitable for acquiring volumetric data and blood vessel flow data, is made by using means suitable for acquiring volumetric data and blood vessel flow data (not shown in the figures).

Just as an example, said means suitable for acquiring volumetric data and blood vessel flow data comprise an echo-doppler or an echo-color doppler, operationally connected to said electronic processor means (102), to said software means (103), and to said means suitable for positioning a human body (104).

In this embodiment of the invention, said means suitable for acquiring volumetric data and blood vessel flow data are suitable for generating morphological indicators and a model of the venous tree of a human body, or a portion thereof.

With a special reference to the lower limbs, said model of the venous tree is advantageously classified on the basis of eleven standard models of the venous tree, or phenotypes. Said phenotypes are a classification of various typologies of chronic venous insufficiency of the lower limbs, a pathological situation wherein an increase in the volume of the limbs takes place, related to a deficiency of the drainage system constituted by veins.

It was experimentally confirmed that relating the volumetric indices to the venous hemodynamic data (analyzing the blood flow in the veins of the lower limbs), as obtained by using the software means 103 suitable for characterizing the various morpho-functional typologies of said venous system, leads to a diagnostic classification of the pathologies of the lower limbs, based on objective data, hence processable in the mentioned models of the venous tree.

Analyzing said volumetric and perimetric indicators, generated at every clinical test with the model of the venous tree, over time, allows an accurate monitoring of pathologies falling in the group comprising muscle, cardiovascular, venous system, and lymphatic system pathologies.

For example, said monitoring over time is made on edemas and pathologies of the lower limbs.

According to the present embodiment, the basic model of the venous tree of the lower limbs is semi-automatically performed and classified by said software means (103) in a specific category of phenotypes and/or sub-categories thereof, referable to volumetric data and blood vessel flow data.

Here follows an exemplary monitoring of volumetric indices in relation to the progress of a pathology of the lower limbs.

### Example 1.

In one first example, the diagnostic system and its respective method was applied for assessing and monitoring volumetric and perimetric indicators of the lower limbs 402 and 403. Said diagnostic system 101 was configured as follow:
- Means suitable for assessing volumetric and perimetric data comprising: four color HD Cameras 1920x1080 pixels - 30Hz; four infrared depth sensors 640x480 pixels - 30Hz with a visual field of 85° and an acquisition distance ranging from 0.2 to 1.5 meters; three movable laser lines.
- Lighting means comprising four 27W LED spotlights for optimizing photographic acquisition.
- Electronic processor means comprising a computer.
- Software means generating volumetric and perimetric indicators and providing a relation of said indicators to the tridimensional representation of the venous hemodynamic map, i.e. the phenotypes.
- Means for positioning a human body comprising a footboard (having a dimension equal to 80 cm, the arms being closed; equal to 170 cm, the arms being open), and a handle with a maximum height equal to 140 cm.

The volume of the lower limb to be assessed and monitored was: diameter: variable; height: 100 cm.

Three markers positioned at the groin, femoral condyles, and malleolus-ankle levels respectively have been used to apply the method.

The diagnostic system according to the present invention, and its respective method, made it possible to generate the following volumetric indicators for the first lower limb, for instance, as an average of several measurements:
- Global volumetric indicator: 10,561.01 cc;
- Ist quartile volumetric indicator: 2,813.32 cc;
- IInd quartile volumetric indicator: 2,040.43 cc;
- Illrd quartile volumetric indicator: 1,759.99 cc
- IVth quartile volumetric indicator: 553.63 cc.

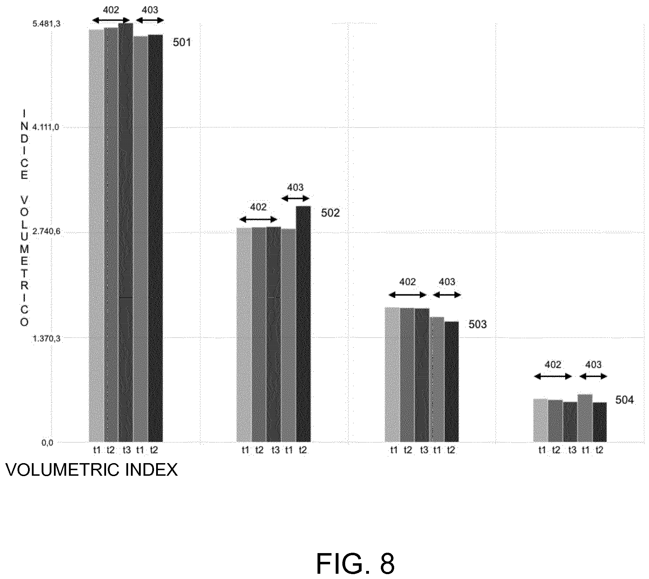

Figure 8 shows how the individual volumetric indicators 501, 502, 503 and 504 for the two lower limbs 402 and 403, whose variation is related to the pathological status of the lower limbs being analyzed, vary as a function of time (t1, t2, t3).

The foregoing demonstrates that the present invention allows to achieve the objects presented in the introduction in an easy and advantageous manner.

The invention is defined by the here attached claims.

## Claims

1. A diagnostic system (100) for assessing and monitoring volumetric indicators and perimetric indicators of a human body, or portions thereof, comprising:
- means suitable for assessing volumetric and perimetric data (101),
- electronic processor means (102) and software means (103) operationally connected to said means suitable for assessing volumetric and perimetric data (101), to process said volumetric and perimetric data generating volumetric and perimetric indicators,
- means for positioning a human body (104) operationally connected to said means suitable for assessing volumetric and perimetric data (101),
- means for lighting a human body (105) connected to said means for positioning a human body (104) and operationally connected to said means suitable for assessing volumetric and perimetric data (101) and to said electronic processor means (102),
**characterized in that** said means suitable for assessing volumetric data and perimetric data (101) are means for acquiring images comprising, video cameras (202) integrally connected to the means for positioning a human body (104), being connected to a footboard (207), via folding movable arms (203), at junction points (204), wherein every arm (203) accommodates one or more video cameras (202).

2. The diagnostic system according to claim 1 **characterized in that** said video cameras (202) are four video cameras 202 positioned in a circular manner about the lower limb.

3. The diagnostic system according to claim 1 **characterized in that** said video cameras (202) include one or several depth sensors.

4. The diagnostic system according to claim 3 **characterized in that** said one or several depth sensors are infrared sensors.

5. The diagnostic system according to one or several of the previous claims **characterized in that** said means suitable for assessing and monitoring volumetric indicators and perimetric indicators (101) comprise both video cameras (202) and an echo-doppler, said video cameras (202) including one or more infrared depth sensors.

6. The diagnostic system according to claim 5 **characterized in that** said echo- doppler acquires volumetric data and blood vessel flow data, and is suitable for determining the typology of the venous tree of the lower limbs.

## Patentansprüche

1. Diagnosesystem (100) zur Auswertung und Überwachung von volumetrischen Indikatoren und perimetrischen Indikatoren eines menschlichen Körpers oder Teilen davon, umfassend:
- Mittel zur Auswertung von volumetrischen und perimetrischen (101),
- elektronische Verarbeitungsmittel (102) und Softwaremittel (103), die operativ mit den genannten für die Auswertung von volumetrischen und perimetrischen Daten (101) geeigneten Mitteln verbunden sind, für die Verarbeitung der genannten volumetrischen und perimetrischen Daten mit Erzeugung von volumetrischen und perimetrischen Indikatoren,
- Mittel zur Positionierung eines menschlichen Körpers (104), die operativ mit den für die Auswertung von volumetrischen und perimetrischen Daten (101) geeigneten Mitteln verbunden sind,
- Mittel zur Beleuchtung eines menschlichen Körpers (105), die mit den Mitteln zur Positionierung eines menschlichen Körpers (104) verbunden und operativ mit den Mitteln verbunden sind, die für die Auswertung von volumetrischen und perimetrischen Daten (101) und mit den Mitteln zur elektronischen Verarbeitung (102) geeignet sind,
**dadurch gekennzeichnet, dass** die zur Auswertung von volumetrischen und perimetrischen Daten geeigneten Mittel (101) Bilderfassungsmittel sind, die Videokameras (202) umfassen, die integral mit den Mitteln zur Positionierung eines menschlichen Körpers (104) verbunden sind und mit einer Stufe (207) durch klappbare bewegliche Arme (203) an Verbindungspunkten (204) verbunden sind, in denen jeder Arm (203) eine oder mehrere Videokameras (202) aufnimmt.

2. Diagnosesystem nach Anspruch 1, **dadurch gekennzeichnet, dass** es sich bei den Videokameras (202) um vier Videokameras 202 handelt, die kreisförmig um die untere Extremität herum positioniert sind.

3. Diagnosesystem nach Anspruch 1, **dadurch gekennzeichnet, dass** die Videokameras (202) einen oder mehrere Tiefensensoren umfassen.

4. Diagnosesystem nach Anspruch 3, **dadurch gekennzeichnet, dass** einer oder mehrere der Tiefensensoren Infrarotsensoren sind.

5. Diagnosesystem nach einem oder mehreren der vorhergehenden Ansprüche, **dadurch gekennzeichnet, dass** die Mittel zur Auswertung und Überwachung von volumetrischen Indikatoren und perimetrischen Indikatoren (101) sowohl Videokameras (202) als auch einen Echo-Doppler umfassen, wobei die Videokameras (202) einen oder mehrere Infrarot-Tiefensensoren umfassen.

6. Diagnosesystem nach Anspruch 5, **dadurch gekennzeichnet, dass** der Doppler-Ultraschall volumetrische Daten und Blutflussdaten erfasst und in der Lage ist, die Typologie des Venenbaums der unteren Extremitäten zu bestimmen.

## Revendications

1. Système de diagnostic (100) pour évaluer et surveiller des indicateurs volumétriques et des indicateurs périmétriques d'un corps humain, ou de parties de celui-ci, comprenant:
- des moyens adaptés à l'évaluation de données volumétriques et périmétriques (101),
- des moyens de traitement électronique (102) et des moyens logiciels (103) reliés de manière opérationnelle auxdits moyens adaptés à l'évaluation de données volumétriques et périmétriques (101), pour l'élaboration desdites données volumétriques et périmétriques avec génération d'indicateurs volumétriques et périmétriques,
- des moyens de positionnement d'un corps humain (104) reliés de manière opérationnelle auxdits moyens adaptés à l'évaluation de données volumétriques et périmétriques (101),
- des moyens d'éclairage d'un corps humain (105) reliés auxdits moyens de positionnement d'un corps humain (104) et reliés de manière opérationnelle auxdits moyens adaptés à l'évaluation de données volumétriques et périmétriques (101) et auxdits moyens de traitement électronique (102),
**caractérisé en ce que** lesdits moyens adaptés à l'évaluation de données volumétriques et périmétriques (101) sont des moyens d'acquisition d'images comprenant des caméras vidéo (202) intégralement reliées aux moyens pour positionner un corps humain (104), étant reliés à un marchepied (207), par des bras mobiles pliables (203), au niveau de points de jonction (204), dans lequel chaque bras (203) reçoit une ou plusieurs caméras vidéo (202).

2. Système de diagnostic selon la revendication 1 **caractérisé en ce que** lesdites caméras vidéo (202) sont quatre caméras vidéo 202 positionnées en manière circulaire autour de l'extrémité inférieure.

3. Système de diagnostic selon la revendication 1 **caractérisé en ce que** lesdites caméras vidéo (202) comprennent un ou plusieurs capteurs de profondeur.

4. Système de diagnostic selon la revendication 3 **caractérisé en ce que** un ou plusieurs desdits capteurs de profondeur sont des capteurs infrarouges.

5. Système de diagnostic selon l'une ou plusieurs des revendications précédentes, **caractérisé en ce que** lesdits moyens aptes à évaluer et à surveiller des indicateurs volumétriques et des indicateurs périmétriques (101) comprennent à la fois des caméras vidéo (202) et un écho-doppler, lesdites caméras vidéo (202) comprenant un ou plusieurs capteurs de profondeur infrarouges.

6. Système de diagnostic selon la revendication 5, **caractérisé en ce que** ledit écho-doppler acquiert des données volumétriques et des données de flux sanguin, et est apte à déterminer la typologie de l'arbre veineux des membres inférieurs.
